# EUROPEAN PATENT APPLICATION

(11) **EP 0 819 768 A2**
(43) Date of publication of application: **21.01.1998**
(21) Application number: 97112013.4
(22) Date of filing: 15.07.1997
(51) Int. Cl.: C12Q 1/68

(54) **Thermophilic strand displacement amplification using AvaI**

(30) Priority: 17.07.1996 US 665996
(71) Applicant: Becton, Dickinson and Company, Franklin Lakes, New Jersey 07417-1880 (US)
(72) Inventor: Pearson, Robert E., Durham, North Carolina 27713 (US)
(74) Representative: Rambelli, Paolo

(57) **Abstract**

The mesophilic restriction endonuclease AvaI and its isoschizomers derived from *Anabaena* and *Anabaenopsis* have been found to support highly efficient amplification of target nucleic acid sequences in thermophilic Strand Displacement Amplification (tSDA). The maximum amplification factors observed occurred at temperatures which were previously reported to result in reduced enzyme activity. As AvaI is an isoschizomer of BsoBI (a thermophilic restriction endonuclease commonly used in tSDA), the invention provides an alternative enzyme which is more readily available than BsoBI and does not require the redesign of SDA amplification primers previously constructed for use in the BsoBI system.

## Description

### FIELD OF THE INVENTION

The present invention relates to methods for amplifying nucleic acid sequences.

### BACKGROUND OF THE INVENTION

*In vitro* nucleic acid amplification techniques have provided powerful tools for detection and analysis of small amounts of nucleic acids. The extreme sensitivity of such methods has lead to attempts to develop them for diagnosis of infectious and genetic diseases, isolation of genes for analysis, and detection of specific nucleic acids as in forensic medicine. Methods such as Strand Displacement Amplification (SDA; G. T. Walker, et al. 1992. *Proc*. *Natl. Acad. Sci. USA* **89**, 392-396; G. T. Walker, et al. 1992. *Nuc. Acids. Res*. **20**, 1691-1696; U.S. Patent No. 5,455,166; U.S. Patent No. 5,270,184; EP 0 684 315) are isothermal reactions which are conducted at a constant temperature.

The SDA reaction initially developed ("conventional SDA") was conducted at a constant temperature between about 37°C and 42°C (U.S. Patent No. 5,455,166) because either the DNA polymerase or the restriction endonuclease (e.g., HincII) or both were mesoptilic enzymes which are thermolabile (temperature sensitive) at temperatures above this range. The enzymes which drive the amplification are therefore inactivated as the reaction temperature is increased above about 42°C. SDA methods which could be performed in a higher temperature range than conventional SDA (about 50°C to 70°C, "thermophilic SDA") were later developed. Thermophilic SDA (tSDA) is described in published European Patent Application No. 0 684 315 and employs 1) thermophilic restriction endonucleases which nick the hemimodified restriction endonuclease recognition/cleavage site at higher temperatures and 2) thermophilic polymerases which extend from the nick and displace the downstream strand in the same temperature range as the restriction endonuclease. At the increased temperatures of tSDA, the amplification reaction has improved specificity, improved efficiency and reduced nonspecific background amplification which often results in improved yields of amplification products. In addition, the need to add the enzymes in a separate step after the initial heat denaturation of double stranded targets may be eliminated when enzymes which are stable at the denaturation temperature are used. UDG decontamination of target-specific amplicons in the SDA reaction is also more efficient when the amount of nonspecific background amplicons is reduced.

The SDA reaction is described in U.S. Patent No. 5,455,166, U.S. Patent No. 5,270,184 and EP 0 684 315. The steps of the SDA reaction are the same regardless of the temperature and enzymes employed, and it requires several specific enzymatic activities in order to successfully amplify a target sequence. Further, SDA requires that nicking by the restriction endonuclease and polymerization/strand displacement by the polymerase occur concurrently within the same temperature range. The two enzymes must have temperature and reaction requirements for these activities which are compatible with each other and with SDA in order for both to function efficiently in the same SDA reaction mix. The SDA polymerase must 1) lack 5'-3' exonuclease activity, either naturally or by inactivation, 2) incorporate the derivatized nucleotides required by SDA (nucleotide analogs such as αthio-dNTPs or other modified dNTPs), 3) displace a downsteam single strand from a double stranded molecule starting at a single stranded nick, and preferably 4) incorporate dUTP to allow amplicon decontamination. Examples of thermophilic polymerases having the appropriate biological activities at the temperatures of thermophilic SDA are described in EP 0 684 315.

The SDA restriction endonuclease must 1) nick (i.e., cleave a single strand of) its double stranded recognition/cleavage site when the recognition/cleavage site is hemimodified, 2) dissociate from its recognition/cleavage site to allow the polymerase to bind and amplify the target, and preferably 3) be unaffected by dUTP incorporated into its recognition/cleavage site. The restriction endonuclease must exhibit these activities under temperature and reaction conditions compatible with expression of the activities required of the polymerase.

Terms relating to amplification by SDA are defined in EP 0 684 315.

AvaI is a restriction endonuclease derived from *Anabaena variabilis* which recognizes and cleaves the sequence:
^{5'} CPyCGPuG³'
³' GPuGCPyC⁵'
Many isoschizomers of AvaI are also known, including BsoBI, AquI, BcoI, Eco88I, NspIII, Ama87I, AcrI, AspBI, AspCI, AspDI, AvrI, Bse15T, Bst7QI, BstSI, BsZ4I, Eco27kI, EspHK9I, Nli387/7I, NliI, NmuAI, NspDI, NspEI, NspIII, NspSAI, Uba1205II, Uba1436I, Uba1440I and Umi5I. AvaI, BsoBI, Ama87I, BcoI and Eco88I are commercially available. BsoBI is derived from the thermophilic bacterium *Bacillus stearothermophilus* and is one of the thermophilic restriction endonucleases commonly used in thermophilic SDA reactions. Like AvaI, AspBI, AspCI, AspDI and AvrI are derived from *Anabaena* species. In contrast to *B. stearothermophilus, Anabaena* is a mesophilic organism and the art-recognized temperature range for AvaI activity is also mesophilic. AvaI is the best characterized of the *Anabaena* restriction endonucleases in this group. The recommended temperature for AvaI double-stranded cleavage of its recognition site is about 37°C, although it has been reported that the double-stranded cleavage activity of AvaI is enhanced at 45°C (Life Technologies, Gaithersburg, MD, 1993-1994 Catalogue and Reference Guide, pg. 6-10; Stratagene, LaJolla, CA, 1995 Catalog, pg 211). It has also been reported that maximum double-stranded cleavage activity' is observed at about 50°C, with rapid inactivation above 60°C (Pohl, et al. 1982. *Eur. J. Biochem.* **123**, 141-152). It has therefore been generally accepted that the useful temperature range for AvaI cleavage of DNA does not exceed about 50°C (Pohl, et al., *supra*). Above about 50°C, the enzyme would be expected to exhibit reduced activity with ultimate inactivation. AvaI has previously been identified as a restriction endonuclease which is useful in conventional SDA (U.S. Patent No. 5,455,166 and U.S. Patent No. 5,270,184), i.e., it nicks its hemimodified restriction endonuclease recognition site in the lower temperature range of conventional SDA. Prior to the present invention it was not known whether or not AvaI would exhibit the activities necessary for SDA at temperatures above those of conventional SDA, but, based on its accepted useful temperature range for double-stranded cleavage, it was generally believed that AvaI activity of any kind would be reduced or absent in the temperature range of thermophilic SDA. AcrI is derived from the closely related genus *Anabaenopsis (Anabaenopsis circularis*), which is also a mesophilic organism.

### SUMMARY OF THE INVENTION

It has now unexpectedly been found that AvaI and its isoschizomers derived from *Anabaena* species or *Anabaenopsis* species, at temperatures compatible with thermophilic SDA, exhibit the activities required for SDA and enhance amplification efficiency. These reaction temperatures are above the previously recognized temperature optimum for these enzymes and in the range where reduced enzyme activity would be predicted from the prior art. The optimum temperature range for AvaI in SDA has been found to be about 47-57°C, with maximum amplification occurring at about 50-55°C.

### DESCRIPTION OF THE DRAWINGS

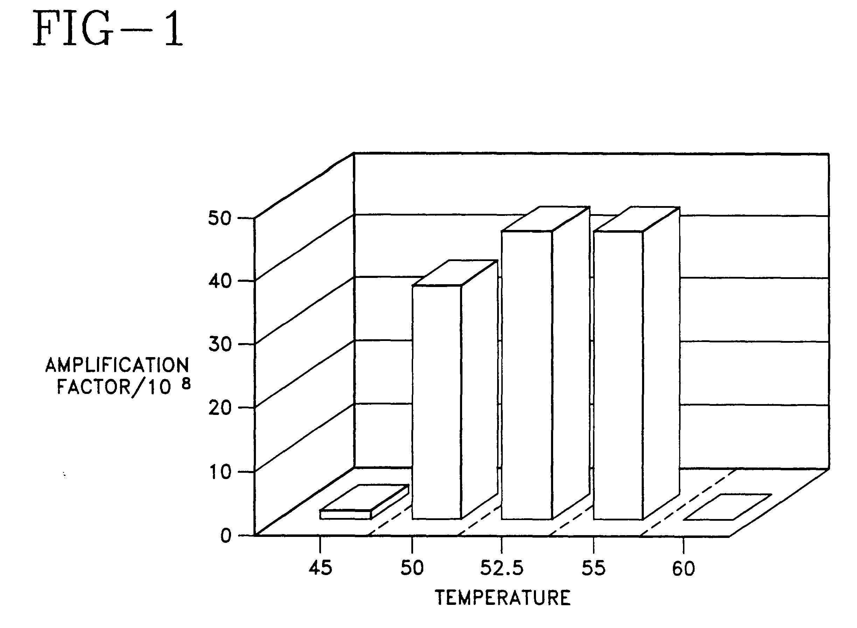

Fig. 1 illustrates amplification factors obtained at various temperatures in tSDA reactions using AvaI.

### DETAILED DESCRIPTION OF THE INVENTION

AvaI was tested in thermophilic SDA (tSDA) to determine whether or not this restriction endonuclease exhibits nicking activity at temperatures above its recognized optimum for double-stranded cleavage and to evaluate its utility in tSDA reactions. AvaI has previously been shown to exhibit the necessary nicking activity at the lower temperatures of conventional SDA which correspond to the art-recognized temperature optimum for this enzyme. Although it was previously believed that AvaI is rapidly inactivated at temperatures above about 50°C, it was unexpectedly found the efficiency of the SDA reaction is enhanced between about 47°C and 57°C when AvaI is the restriction endonuclease.

The tSDA reaction was performed essentially as described in EP 0 684 315, substituting AvaI as the restriction endonuclease. Conventional SDA reactions were performed essentially as described in U.S. Patent No. 5,435,166 and U.S. Patent No. 5,270,184. A pUC 19 vector with a primer-cassette sequence inserted between the EcoRI and HindIII sites provided the target sequence. The primer cassette contained the sequences for hybridization of the amplification and bumper primers:

### Amplification Primers (target binding sequences italicized, AvaI sites bolded)

- S1.1: ACCGCATCGAATGCATGT**CTCGGG***TAAGGCGTACTCGACC* (SEQ ID NO:1)
- S2.2: CGATTCCGCTCCAGACTT**CTCGGG***TGTACTGAGATCCCCT* (SEQ ID NO:2)

### Bumper Primers

- B1: CGATCGAGCAAGCCA (SEQ ID N0:3)
- B2: CGAGCCGCTCGCTGA (SEQ ID NO:4)
Amplification of the target region between the primer binding sites in the pUD 19 vector produced a 70 base pair amplicon. The target sequence of the EcoRI-linearized vector (10³ molecules/reaction) was amplified in 0.1 mg/ml BSA, 25 mM potassium phosphate, 1.4 mM α-thio dCTP, 0.5 mM dUTP, 0.2 mM dATP, 0.2 mM dGTP, 0.5 µM each amplification primer, 0.05 µM each bumper primer, 10% glycerol, 5 mM magnesium acetate, 500 ng human placental DNA, 150 units AvaI and 18 units exo⁻ Bst polymerase or 50 units exo⁻ Klenow. Exo⁻ Klenow was the polymerase used in lower temperature reactions (37°C and 40°C) and Bst was the polymerase used at higher temperatures (45-60°C). Control reactions contained no target DNA. Reactions containing all components except the enzymes were boiled for 3 min. and transferred to a heating block at the temperature selected for amplification (37°C, 40°C, 45°C, 50°C, 52.5°C, 55°C or 60°C) where they were equilibrated for 4 min. The enzymes were added and the amplification reaction was allowed to proceed for 30 min. The reactions were stopped by boiling for 5 min. Amplification products were detected in a 5 µl aliquot of each reaction by hybridization and extension of a ³²P-labeled probe at 37°C, essentially as described by Walker, et al. (1992. *PNAS, supra*). The probe extension products were visualized by electrophoresis on an 8% denaturing gel.

The results are shown in the following Table and illustrated graphically in Fig. 1:

| AMPLIFICATION TEMPERATURE | AMPLIFICATION FACTOR |
|---|---|
| 37°C | NA |
| 40°C | NA |
| 45°C | 1 X 10⁸ |
| 50°C | 3.7 X 10⁹ |
| 52.5°C | 4.4 X 10⁹ |
| 55°C | 4.4 X 10⁹ |
| 60°C | 1 X 10⁷ |

Amplification was observed between about 45°C and 60°C, with maximum efficiency at about 50-55°C. Between 55°C and 60°C there was a rapid decrease in amplification efficiency, however, these results suggest that highly efficient amplification extends to temperatures slightly above 55°C (probably up to about 57°C) and to temperatures slightly below 50°C (probably as low as about 47°C). The preferred temperature range for amplification using AvaI is therefore estimated to be about 47-57°C.

No amplification products were visualized on the gel for amplification reactions performed at 40°C or 37°C. This is most likely a result of the 30 min. reaction time, as amplification is much slower at the lower temperatures of conventional SDA. Conventional SDA reactions are typically allowed to proceed for two hours. Thirty minutes at conventional SDA temperatures is probably insufficient to produce an amount of amplification product which can be detected by gel electrophoresis and short periods of autoradiography, particular considering the exponential nature of amplification reaction.

The present invention is based on the discovery that the mesophilic restriction endonucleases which recognize and cleave the sequence
^{5'} CPyCGPuG^{3'}
^{3'} GPuGCPyC^{5'}
(AvaI and its isoschizomers derived from *Anabaena* species and *Anabaenopsis* species) support highly efficient tSDA, providing amplification efficiencies which are approximately equivalent to the thermophilic restriction endonucleases (e.g., BsoBI) typically selected for these reactions because of their recognized temperature stability. AvaI has the advantage of being more readily available and less expensive than BsoBI, and its substitution for BsoBI in tSDA does not necessitate redesign of amplification primers previously developed for the BsoBI system (i.e., the enzyme recognition site is the same). The discovery that this restriction endonuclease performs very efficiently at the temperatures of tSDA allows the practitioner to take advantage of the improved performance of tSDA without incurring the increased cost of less commonly used enzymes such as BsoBI. Isoschizomers of AvaI derived from *Anabaena variabilis* (e.g., AvrI), from species of *Anabaena* (e.g., AspBI, AspCI and AspDI) and from the closely related genus *Anabaenopsis* (e.g., AcrI) would be expected to have similar properties as regards tSDA. If it is not commercially available, the selected isoschizomer may be isolated from the organism as described by M. G. C. Duyvesteyn, et al. (1983. *Arch. Microbiol*. **134**, 276-281). The quantities of the selected restriction endonuclease used in the tSDA reaction are not critical, nor are the particular target to be amplified or the selected primers. One skilled in the art may routinely adjust these parameters and the time, temperature and concentration of reactants to obtain the desired result as described in the literature for SDA. The foregoing example is provided merely to illustrate one embodiment of the invention and is not to be construed as limiting the scope of the invention defined by the appended claims.

## Claims

1. A method for amplifying a target nucleic acid sequence comprising amplifying the target nucleic acid sequence at about 45-60°C in a Strand Displacement Amplification reaction comprising a restriction endonuclease selected from the group consisting of AvaI, isochizomers of AvaI derived from *Anabaena* and isoschizomers of AvaI derived from *Anabaenopsis.*

2. The method of Claim 1 wherein the restriction endonuclease is selected from the group consisting of AvaI, AspBI, AspCI, AspDI, AvrI and AcrI.

3. The method of Claim 1 wherein the target nucleic acid sequence is amplified at about 47-57°C.

4. The method of Claim 3 wherein the target nucleic acid sequence is amplified at about 50-55°C.

5. The method of Claim 3 wherein the target nucleic acid sequence is amplified for about 30 min.

6. The method of Claim 1 further comprising detecting the amplified target nucleic acid sequence.

7. The method of Claim 6 wherein the amplified target nucleic acid sequence is detected by hybridization of a labeled oligonucleotide probe.


SEQUENCE LISTING

(1) GENERAL INFORMATION:

(i) APPLICANT: BECTON, DICKINSON AND COMPANY
(A) NAME: BECTON, DICKINSON AND COMPANY
(B) STREET: 1 Becton Drive
(C) CITY: Franklin Lakes
(D) STATE: NEW JERSEY
(E) COUNTRY: USA
(F) ZIP: 07417

(ii) TITLE OF INVENTION: THERMOPHILIC STRAND DISPLACEMENT
AMPLIFICATION USING AvaI

(iii) NUMBER OF SEQUENCES: 4

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30

(v) CURRENT APPLICATION DATA:
(A) APPLICATION NUMBER:
(B) FILING DATE:

(2) INFORMATION FOR SEQ ID NO:1:

(i) SEQUENCE CHARACTERISTICS:
(A) LENGTH: 40 base pairs
(B) TYPE: nucleic acid
(C) STRANDEDNESS: single
(D) TOPOLOGY: linear

(xi) SEQUENCE DESCRIPTION: SEQ ID NO:1:

ACCGCATCGA ATGCATGTCT CGGGTAAGGC GTACTCGACC 40

(2) INFORMATION FOR SEQ ID NO:2:

(i) SEQUENCE CHARACTERISTICS:
(A) LENGTH: 40 base pairs
(B) TYPE: nucleic acid
(C) STRANDEDNESS: single

(D) TOPOLOGY: linear

(xi) SEQUENCE DESCRIPTION: SEQ ID NO:2:

CGATTCCGCT CCAGACTTCT CGGGTGTACT GAGATCCCCT 40

(2) INFORMATION FOR SEQ ID NO:3:

(i) SEQUENCE CHARACTERISTICS:
(A) LENGTH: 15 base pairs
(B) TYPE: nucleic acid
(C) STRANDEDNESS: single
(D) TOPOLOGY: linear

(xi) SEQUENCE DESCRIPTION: SEQ ID NO:3:

CGATCGAGCA AGCCA 15

(2) INFORMATION FOR SEQ ID NO:4:

(i) SEQUENCE CHARACTERISTICS:
(A) LENGTH: 15 base pairs
(B) TYPE: nucleic acid
(C) STRANDEDNESS: single
(D) TOPOLOGY: linear

(xi) SEQUENCE DESCRIPTION: SEQ ID NO:4:

CGAGCCGCTC GCTGA 15